# EUROPEAN PATENT APPLICATION

(11) **EP 3 847 903 A1**
(43) Date of publication of application: **14.07.2021**
(21) Application number: 20214658.5
(22) Date of filing: 16.12.2020
(51) Int. Cl.: A23L 7/17, A21D 8/04, A23P 30/34, C12N 9/82, A23L 5/20

(54) **METHOD FOR THE PRODUCTION OF PUFFED PRODUCTS WITH REDUCED ACRYLAMIDE CONTENT**

(30) Priority: 30.12.2019 SK 2032019 U
(71) Applicant: Národné Polnohospodárske a Potravinárske Centrum, 951 41 Luzianky (SK); CELPO spol. s r.o., 962 23 Ocová (SK); Centrum Vedecko Technickych Informácii Srl., 811 04 Bratislava (SK)
(72) Inventor: Ciesarová, Zuzana, 821 02 Bratislava (SK); Murín, Jozef, 962 12 Detva (SK); Kukurová, Kristína, 821 09 Bratislava (SK); Jelemenská, Viera, 900 01 Modra (SK)
(74) Representative: Litváková a spol., s.r.o.

(57) **Abstract**

A method for the production of puffed products with reduced acrylamide content from pellets formed by extrusion of raw material characterized by adding the aqueous solution of asparaginase enzyme to the raw material before the step of extrusion of the raw material. Minimum amount of enzyme used to treat 1 kg of the raw material is 2000 asparaginase units and the moisture of the raw material is at least 25%.

## Description

### Technical Field

The invention belongs to the field of food technology and specifically relates to the method of production of puffed products, especially breads with reduced acrylamide content.

### Background Art

Puffing is a technological process in which grain (or extruded pellets made of grain) is pressed in the matrix of a puffing machine at high pressure (9-12 MPa) and high temperature (220-260°C) for several seconds (3-9 s) and the subsequent sharp drop in pressure in the matrix causes the water to evaporate rapidly, thereby changing the grain structure. The cell nuclei rupture and the volume of the expanded grain increases. Puffing gives the grain a pleasant taste and makes it more digestible.

The starting raw material for the production of puffed breads is flour with a moisture content of 8-12.5% (graham rye wholemeal flour, spelt wholemeal flour 0.2 mm, oat plain flour). The technological process of processing this flour into breads consists of several steps:
- The flour is processed on an extruder, where it is moistened to a moisture content of approximately 21-24%. The moistening takes place directly in the feeder of the extruder for a few seconds before the actual compression and heating in the extruder. The resulting extruded cake passes through a matrix, where it is cut into balls - pellets with a diameter of 0.5-1.0 cm.
- The pellets are dried to moisture content according to the method of further handling on equipment designed for this purpose: for direct processing to a final moisture content of 16-17%, for longer-term storage to a final moisture content of up to 13%.
- The pellets are processed on puffing machines (pressure 90-120 bar, temperature 220-260 °C, baking time 3-6 seconds depending on the type of raw material) into breads with a moisture content of 4%.

With this technology, as in other heat-treated cereal products, the formation of undesirable acrylamide occurs intensively in puffed products. Acrylamide is classified as a probable carcinogen in a category 2A by the International Agency for Research on Cancer (IARC) [1] and is covered by the EU Commission Regulation 2017/2158 [2] establishing mitigation measures and benchmark levels for the reduction of the presence of acrylamide in food. Under this regulation, all food business operators are required to monitor the acrylamide content in their products. If the relevant benchmark level is exceeded, operators are obliged to take mitigation measures to reduce the formation of acrylamide in these products and to redetermine the acrylamide content therein in order to determine the effectiveness of the measures taken.

Acrylamide is not added to food, neither is the food contaminated by it due to the external environment. Based on scientific knowledge, it has been found that acrylamide is formed mainly from compounds that are a natural part of plant material, namely saccharides and the non-essential free amino acid L-asparagine [3-5]. The reaction of these precursors occurs due to the heat treatment of the raw materials at temperatures usually higher than 120°C, while acrylamide is a minor by-product of the ongoing Maillard reaction.

Said precursors are abundantly present in cereal and pseudo-cereal grains, as well as in flour made from them, in raw potatoes, in certain vegetables and fruits, and in coffee beans. The acrylamide content in the final products thus depends on the presence and availability of both precursors in the raw materials, on the intensity of heat treatment and on other factors that affect the extent of Maillard reaction (pH, moisture, presence of various inorganic and organic salts, especially builders or additives etc.). By changing these parameters, it is possible to affect the formation of acrylamide, although its complete elimination is not possible. Thus, the limiting factor for acrylamide formation is the content of reducing saccharides for potato products and the content of asparagine for cereal products.

In the case of puffed breads, our measured values of acrylamide ranged from 36 to 772 µg/kg depending on the type of flour used (Table 1), while the benchmark level for this type of product given in the EU Commission Regulation no. 2017/2158 is at the level of 300 µg/kg. This means that in the case of oat, wheat, spelt and rye breads, it is necessary to apply some of the measures to reduce the amount of acrylamide directly in the production.

**Table 1 Acrylamide content in wholemeal puffed breads**

| **Type of bread** | **Acrylamide (µg/kg)** |
|---|---|
| Buckwheat | 36 ± 7 |
| Organic maize | 114 ± 14 |
| Rice | 224 ± 22 |
| Oat | 363 ± 6 |
| Wheat | 549 ± 42 |
| Organic spelt | 769 ± 98 |
| Organic rye | 772 ± 6 |

The measures by which the acrylamide content can be reduced vary. The most common are the selection of raw materials with reduced content of reducing saccharides and asparagine, addition of minor components (other amino acids, calcium salts), more acidic pH, dilution, sample size, fermentation, setting of baking production conditions, especially in terms of temperature, baking time and moisture, addition of other technological steps such as washing, blanching, addition of divalent salts, product reformulation, change of raising agents, etc. A summary of these measures, which are applicable in individual commodities of food production, is available on the one hand in the relevant EU Commission Regulation no. 2017/2158 [2], on the other hand in the updated Acrylamide Toolbox 2019 manual [6].

In the production of puffed breads, the choice of measures is considerably limited due to the production specificities of this product. Based on our experience and our own measurements, a decrease in temperature and baking time, an increase in moisture and a simultaneous increase in weight and an increase in individual bread thickness proved to be partially effective, however, it was reflected in poorer product quality indicators (poor cohesion, irregular bread shape, insufficient baking quality, lower crunchiness, increased gumminess, atypical colour, taste and smell of bread). In the case of rye breads, the reduction of the baking temperature by 4°C (original upper/lower matrix temperatures 232/236°C, adjusted temperatures 228/232 °C) and shortening of the baking time (original value 3.7 seconds, adjusted value 3.4 seconds) resulted in a decrease in the acrylamide content from 849 µg/kg to 640 µg/kg, i.e. by 25%. If the thickness and weight of the individual breads were increased in order to reduce the relative surface area, the acrylamide value decreased to 506 µg/kg, i.e. by 40%, but at the same time the temperature and baking time had to be increased to properly bake the product (Tab. 2). Despite these measures, this acrylamide value is still relatively high and exceeds the benchmark level of 300 µg/kg set out in the EU Commission Regulation [2].

**Table 2**

| **Number of pcs /100 g** | **Upper matrix temperature (°C)** | **Lower matrix temperature (°C)** | **Baking time (second)** | **Acrylamide (µg/kg)** |
|---|---|---|---|---|
| 18 | 232 | 236 | 3.7 | 849 |
| 18 | 228 | 232 | 3.4 | 640 |
| 14 | 236 | 240 | 4.0 | 506 |

Other possible measures to reduce the acrylamide content include the application of the enzyme to the raw material, which results in the formation of acrylamide during baking. It is an asparaginase enzyme available under various trade names, the use of which in food technology has been approved in many countries of the European Union. It is a technological enzyme that is deactivated by the baking process and is not present in the final product. The principle of action of this enzyme is that it catalyses the conversion of the amino acid asparagine to aspartic acid, which does not form acrylamide. The undeniable advantage of using an enzyme is that its use does not affect the final quality of the product, so there is no undesired change in the organoleptic properties of the product. The enzyme must be applied to the raw material before baking, and the action of the enzyme must last for some time under suitable incubation conditions (temperature, moisture). A product having a reduced acrylamide content is then prepared from such raw material by baking.

In this way, it is possible to prevent the formation of acrylamide in products prepared from liquid dough (chips, snacks, crackers from potato dough or from flour-containing dough), where contact of the enzyme with the material and penetration of the enzyme to the asparagine itself is allowed.

A problem with the use of this enzyme in puffed bread production technology is the environment, which is not suitable for the enzyme to be sufficiently active. The flour from which the pellets are extruded as an intermediate for the production of breads has a moisture content of 8 to 12.5%. After moistening, it reaches a value of 21-24%. The application of the enzyme in powder form to this matrix is ineffective in terms of reducing the content of asparagine, and consequently also acrylamide in the final products.

This has been published in the literature:
*1.* IARC monographs on the evaluation of the carcinogenic risks of chemicals to humans. Vol. 60. Lyon: International Agency for Research on Cancer, 1994, 560 pp. ISBN 92 832 1260 6*.*
*2.* Commission Regulation (EU) 2017/2158 of 20 November 2017 establishing mitigation measures and benchmark levels for the reduction of the presence of acrylamide in food. Official Journal of the European Union of 21.11.2017, L 304/24-304/44. Available from: https://eur-lex.europa.eu/legal-content/SKI/TXT/PDF/?uri-CELEX:32017R2158&from=EN
*3.* Ciesarová, Z. - Kiss, E. - Kolek, E.: Study of factors affecting acrylamide levels in model systems. Czech Journal of Food Sciences, 24, 2006, pp. 133-137*.*
*4.* Ciesarová, Z. - Kukurová, K. - Bednáriková, A. - Hozlár, P. - Ruckschloss, L'.: Amino acid profile of selected wholegrains important to acrylamide formation in cereal-based products. Chemické Listy 102, 2008, pp. 613-614*.*
*5.* Ciesarová, Z. - Kukurová, K. - Bednáriková, A. - Morales F.J.: Effect of heat treatment and dough formulation on the formation of Maillard reaction products in fine bakery products-benefits and weak points. Journal of Food and Nutrition Research, 48 (1), 2009, pp. 20-30*.*
*6.* Acrylamide Toolbox 2019. https://www.fooddrinkeurope.eu/uploads/publications documents/FoodDrinkEurope Acrylamide _Toolbox_2019.pdf

Document CN 107259358 A discloses processing method of a puffed crab crisp high protein rice cake. The method is characterized in that: asparaginase is added to effectively reduce the content of acrylamide in puffed food and improve the food safety; and extrusion expansion is carried out to eliminate various anti-nutrient substances in soybeans through the action of short-term high temperature, high polymer and pressure conditions on soybean protein, thus improving the digestive absorption ability of human body to protein.

Document CA 2618225 A1 discloses a combination of two or more acrylamide-reducing agents which are added to a fabricated food prior to cooking in order to reduce the formation of acrylamide. The fabricated food product can be, for example, a corn chip or a potato chip. Alternatively, a thermally-processed food, such as a potato chip from a sliced potato, can be contacted with a solution having two or more acrylamide-reducing agents prior to cooking. The acrylamide-reducing agents can include asparaginase, di- and trivalent cations, and various amino acids and free thiols. The acrylamide-reducing agents can be added during milling, dry mix, wet mix, or other admix, so that the agents are present throughout the food product.

Document US 2010062123 A1 discloses a method and system for the direct injection of a concentrated additive. In one aspect, the concentrated additive comprises asparaginase. Because dilution of asparaginase in chlorinated drinking water can reduce the activity of the enzyme thereby making the enzyme less effective the direct injection of the enzyme into a dough can increase the acrylamide reduction in food products.

Document CN 101365340 A discloses methods for reducing the level of asparagine in an asparagine-containing dough food component comprise providing an asparagine-reducing enzyme in combination with at least one asparagine-containing dough food component in a medium, wherein the water activity of the medium is greater than about 0.85, preferably greater than about 0.90 are provided. Methods for reducing the level of acrylamide in a dough-based food product employ a dough food component in which the level of asparagine has been reduced to provide dough-based food products with reduced acrylamide levels.

### Nature of Invention

The method for the production of puffed products, in particular cereals, with reduced acrylamide content according to this technical solution substantially eliminates the above-mentioned shortcomings and comprises the following steps:
- moistening the raw material, which is preferably flour for extrusion,
- flour extrusion,
- preparation of pellets,
- drying of pellets,
- puffing of pellets.

The principle of the invention is that the raw material is treated by asparaginase enzyme, this is carried out in a manner that decreases the content of amino acid asparagine to at most 100 mg/kg, which ensures, under the given production conditions of puffed products, that the required acrylamide content is lower than 300 µg/kg. The invention is realized by introducing a new step in the production technology of puffed products before the step of extrusion of the raw material, namely the treatment of raw material intended for extrusion with asparaginase enzyme by adding the aqueous solution of asparaginase enzyme with temperature between 10°C and 37°C to the raw material. The minimum amount of asparaginase enzyme used to treat 1 kg of the raw material is 2000 asparaginase units.

The raw material can be flour or a loose mixture of flour with another loose substance such as salt, spices, etc.

In order to ensure effective application of the asparaginase enzyme, the flour moisture needs to be increased from the initial 8%-12.5% water content (w/w) within the water-flour mixture to the minimum water content within the water-flour mixture of 25% (w/w), where the step of moistening the flour and treating the flour with the enzyme can be carried out simultaneously by adding at least 1 part of aqueous solution of the asparaginase enzyme to 5 parts of flour.

The aqueous solution of the asparaginase enzyme is prepared by diluting the asparaginase enzyme preparation with water in a ratio that yields aqueous solution of the asparaginase enzyme containing at least 2000 asparaginase units per 1 kg of flour. A ratio of water parts to asparagine preparation parts depends on how many asparaginase units contains the asparaginase preparation in 1 part.

It is preferable to add the aqueous solution of the asparaginase enzyme to the flour evenly while stirring the flour constantly. Subsequently, the asparaginase enzyme is allowed to act on the flour for at least 15 min at a temperature of at least 18°C. The even addition of the aqueous solution of the asparaginase enzyme to the flour can be carried out by sprinkling the flour.

Such enzyme-treated flour can be used to prepare pellets by extrusion under usual conditions. Before baking, the pellets are dried in a desiccation tunnel to the moisture value of 16-17% (w/w) within the water-flour mixture. The pellets are used to make puffed bread in baking machine under usual conditions.

By the method for the production of puffed products according to this invention, an acrylamide content of less than 300 µg/kg of product is achieved in the puffed products.

The method for the production of puffed products according to this invention proposes the production technology for puffed products so that it creates appropriate environment, in which the asparaginase enzyme has a sufficient activity. Application of the asparaginase enzyme causes the conversion of amino acid asparagine to aspartic acid, which does not give rise to acrylamide. Thereby, the objective of this invention is achieved, namely the reduced asparagine content within the flour and consequently reduced acrylamide content in the final puffed products.

### Examples of Embodiments

### Example 1

Whole-grain flour from organic rye with initial moisture of 10.3% (w/w) in the water-flour mixture was treated by aqueous solution of the asparaginase enzyme by adding 20 parts of the aqueous solution of the asparaginase enzyme to 100 parts of the flour under constant stirring. The aqueous solution of the asparaginase enzyme is added evenly to the flour by sprinkling. This step increases the moisture of the mixture of water and the aqueous solution of the asparaginase enzyme and the water content in the water-flour mixture is 25% (w/w). The aqueous solution of the asparaginase enzyme is prepared from the commercial preparation of the asparaginase enzyme with the specified concentration of 3500 asparaginase units per gram by mixing 1 part of the preparation with 9 parts of water with temperature of 12°C. The amount of the enzyme used to treat 1 kg of flour was 70000 asparaginase units. The aqueous solution of the enzyme was allowed to react with the flour for at least 15 min at operating temperature of 23°C under constant stirring. Measurements showed that the asparagine content in organic rye flour dropped from the initial 726 mg/kg to 3 mg/kg, which means the level of asparagine was reduced by 99.6%.

### Example 2

Whole-grain flour from organic rye with initial moisture of 10.3% (w/w) in the water-flour mixture was treated by aqueous solution of the asparaginase enzyme by adding 40 parts of the aqueous solution of the asparaginase enzyme to 100 parts of the flour under constant stirring, which increased the moisture of the mixture to 36% (w/w) in the water-flour mixture. The aqueous solution of the asparaginase enzyme is prepared from the commercial preparation of the asparaginase enzyme with the specified concentration of 3500 asparaginase units per gram by mixing 1 part of the preparation with 19 parts of water with temperature of 12°C. The amount of the enzyme used to treat 1 kg of flour was 70000 asparaginase units. The aqueous solution of the asparaginase enzyme was allowed to act on the flour for at least 15 min at operating temperature of 23°C under constant stirring. Measurements showed that the asparagine content in organic rye flour dropped from the initial 726 mg/kg to 0 mg/kg, which means the level of asparagine was reduced by 100%.

### Example 3

Whole-grain flour from organic rye with initial moisture of 10.3% (w/w) in the water-flour mixture was treated by aqueous solution of the asparaginase enzyme by adding 40 parts of the aqueous solution of the asparaginase enzyme to 100 parts of the flour under constant stirring, which increased the moisture of the mixture to 36% (w/w) in the water-flour mixture. The solution of the asparaginase enzyme is prepared from the commercial preparation of the asparaginase enzyme with the specified concentration of 3500 asparaginase units per gram by mixing 1 part of the preparation with 199 parts of water with temperature of 12°C. The amount of the enzyme used to treat 1 kg of flour was 7000 asparaginase units. The aqueous solution of the asparaginase enzyme was allowed to act on the flour for at least 15 min at operating temperature of 23°C under constant stirring. Measurements showed that the asparagine content in organic rye flour dropped from the initial 726 mg/kg to 8 mg/kg, which means the level of asparagine was reduced by 98%.

### Example 4

Whole-grain flour from organic rye with initial moisture of 10.3% (w/w) in the water-flour mix was treated by aqueous solution of the asparaginase enzyme by adding 20 parts of the aqueous solution of the asparaginase enzyme to 100 parts of the flour under constant stirring, which increased the moisture of the mixture to 25% (w/w) in the water-flour mixture. The solution of the asparaginase enzyme is prepared from the commercial preparation of the asparaginase enzyme with the specified concentration of 3500 asparaginase units per gram by mixing 1 part of the preparation with 199 parts of water with temperature of 12°C. The amount of the enzyme used to treat 1 kg of flour was 3500 asparaginase units. The aqueous solution of the enzyme asparaginase was allowed to act on the flour for at least 15 min at operating temperature of 23°C under constant stirring. Measurements showed that the asparagine content in organic rye flour dropped from the initial 726 mg/kg to 9 mg/kg, which means the level of asparagine was reduced by 98.5%.

### Example 5

Whole-grain flour from organic rye with initial moisture of 10.3% (w/w) in the water-flour mixture was treated by aqueous solution of the asparaginase enzyme by adding 20 parts of the aqueous solution of the asparaginase enzyme to 100 parts of the flour under constant stirring, which increased the moisture of the mixture to 25% (w/w) in the water-flour mixture. The solution of the enzyme is prepared from the commercial preparation of the asparaginase enzyme with the specified concentration of 3500 asparaginase units per gram by mixing 1 part of the preparation with 199 parts of water with temperature of 23°C. The amount of the enzyme used to treat 1 kg of flour was 3500 asparaginase units. The aqueous solution of the enzyme was allowed to act on the flour for at least 15 min at operating temperature of 23°C under constant stirring. Measurements showed that the asparagine content in organic rye flour dropped from the initial 726 mg/kg to 11 mg/kg, which means the level of asparagine was reduced by 98%.

### Example 6

Whole-grain flour from organic rye with initial moisture of 10.3% (w/w) in the water-flour mixture was treated by aqueous solution of the asparaginase enzyme by adding by spreader 20 parts of the aqueous solution of the asparaginase enzyme to 100 parts of the flour under constant stirring for 7 minutes, which increased the moisture of the mixture to 25% (w/w) in the water-flour mixture. The aqueous solution of the asparaginase enzyme is prepared from the commercial preparation of the asparaginase enzyme with the specified concentration of 3500 asparaginase units per gram by mixing 1 part of the preparation with 99 parts of water with temperature of 25°C, in which the salt has been dissolved (50 g/l). The enzyme solution was allowed to activate for 15 minutes at 25°C. The amount of the enzyme used to treat 1 kg of flour was 7000 asparaginase units. After adding an aqueous solution of the asparaginase enzyme, the mixture was stirred for 5 minutes, then allowed to stand at 20°C for 24 hours. Pellets were extruded from the mixture and were dried in a drying tunnel to a moisture value of 16.9%. Measurements showed that the asparagine content in pellets from enzymatically treated organic rye flour dropped from the initial 760 mg/kg to 96 mg/kg, which means the level of asparagine was reduced by 87%.

### Example 7

Whole-grain flour from organic rye with initial moisture of 10.3% (w/w) in the water-flour mixture was treated by aqueous solution of the asparaginase enzyme by adding by spreader 20 parts of the aqueous solution of the asparaginase enzyme to 100 parts of the flour under constant stirring for 7 minutes, which increased the moisture of the mixture to 25% (w/w) in the water-flour mixture. The aqueous solution of the enzyme is prepared from the commercial preparation of the asparaginase enzyme with the specified concentration of 3500 asparaginase units per gram by mixing 1 part of the preparation with 99 parts of water with temperature of 25°C, in which the salt has been dissolved (50 g/l). The aqueous solution of the enzyme was allowed to activate for 15 minutes at 25°C. The amount of the enzyme used to treat 1 kg of flour was 7000 asparaginase units. After adding an aqueous solution of the asparaginase enzyme, the mixture was stirred for 5 minutes, then allowed to stand at 20°C for 24 hours. Pellets were extruded from the mixture and were dried in a drying tunnel to a moisture value of 16.9%. Breads were made from pellets in the usual way. Measurements showed that the asparagine content in breads from enzymatically treated organic rye flour was reduced by 55% compared to breads prepared in the same way from flour without enzyme treatment, i.e. dropped from the initial 660 µg/kg to 300 µg/kg.

### Industrial applicability

The invention is industrially applicable in the food industry, especially in food production plants that deal with extrusion and production of puffed products.

## Claims

1. A method for the production of puffed products with reduced acrylamide content from pellets formed by extrusion of raw material, **characterized in that** before the step of extrusion of the raw material, the raw material is treated with asparaginase enzyme by adding the aqueous solution of asparaginase enzyme with temperature between 10°C and 37°C to the raw material, wherein the minimum amount of enzyme used to treat 1 kg of the raw material is 2000 asparaginase units and the moisture of the raw material is at least 25%.

2. The method for the production of puffed products according to claim 1, **characterized in that** the aqueous solution of asparaginase enzyme is prepared by diluting the asparaginase enzyme preparation with water in such a ratio as to obtain an aqueous asparaginase enzyme solution containing at least 2000 asparaginase units per 1 kg of flour.

3. The method for the production of puffed products according to any one of claims 1 and 2, **characterized in that** the raw material is flour or a loose mixture of flour with another loose substance.

4. The method for the production of puffed products according to any one of claims 1 to 3, **characterized in that** the aqueous solution of the asparaginase enzyme is added evenly to the raw material while stirring constantly and the asparaginase enzyme is allowed to act on the raw material for at least 15 minutes at a temperature of at least 18°C.

5. The method for the production of puffed products according to any one of claims 1 to 4, **characterized in that** the even addition of the aqueous solution of the asparaginase enzyme is carried out by sprinkling the raw material.

6. The method for the production of puffed products according to any one of claims 1 to 5, **characterized in that** the step of moistening the raw material and treating the raw material with an asparaginase enzyme is carried out simultaneously by adding at least 1 part of aqueous enzyme solution to 5 parts of the raw material.
